# EUROPÄISCHE PATENTSCHRIFT

(11) **EP 0 404 868 B1**
(45) Veröffentlichungstag und Bekanntmachung des Hinweises auf die Patenterteilung: **27.10.1993**
(21) Anmeldenummer: 89909185.4
(22) Anmeldetag: 12.08.1989
(51) Int. Cl.: A61K 7/13

(54) **VERFAHREN ZUM FÄRBEN VON HAAREN MIT PFLANZENFARBEN**
PROCESS FOR DYEING HAIR WITH VEGETABLE DYES
PROCEDE POUR TEINDRE LES CHEVEUX AVEC DES COLORANTS D'ORIGINE VEGETALE

(30) Priorität: 27.08.1988 DE 3829102
(43) Veröffentlichungstag der Anmeldung: 02.01.1991
(73) Patentinhaber: Wella Aktiengesellschaft, 64295 Darmstadt (DE)
(72) Erfinder: BAUER, Rudolf, D-7000 Stuttgart (DE)
(86) Internationale Anmeldenummer: EP8900960
(87) Internationale Veröffentlichungsnummer: WO9001922

(56) Entgegenhaltungen:
- FR-A- 1 173 973
- GB-A- 2 093 868
- Patent Abstracts of Japan, Band 10, Nr. 339 (C-385)(2395), 15. November 1986, & JP-A-61143315 (nippon Nachiyuberu K.K.) 1. Juli 1986
- International Journal of Cosmetic Science, Band 4, 1982, International Journal of Cosmetic Science, J.P. Forestier: "Henné, Absorption de la lawsone par le cheveu"
- Römpps Chemic-Lexikon, 8. Aufl., Franckh'sche Verlagshandlung, Seiten 1674 und 1808

## Beschreibung

Die Erfindung betrifft ein Verfahren zum Färben Von Haaren.

Man wünscht heute immer mehr die Verwendung von Pflanzenfarben beim Färben von Haaren, um die nachteiligen Auswirkungen der ansonsten verwendeten Oxidationsmittel zu vermeiden, da einige dieser Substanzen unter dem Verdacht gesundheitsschädlicher insbesondere carcinogener Wirkung stehen oder bei empfindlichen Personen starke allergische Reaktionen hervorrufen (vgl. die VO über kosmetische Mittel vom 16.12.1977, BGBl. I. S. 2589).

Andererseits besteht ein Nachteil des bekannten Färbens von Haaren mit Pflanzenfarben darin, daß sich mit ihnen nur bestimmte Nuancen (blond, braun, rötlich, aschfarben usw.) erzielen lassen, die Verfärbung der Cuticula durch die aufgebrachten Pflanzenfarben hat gleichsam nur die Wirkung eines Farbfilters für die natürliche Eigenfarbe der darunter befindlichen Kortex. Ergrautes Haar, das keine natürlichen Pigmente mehr aufweist, wird nicht abgedeckt, da die unter der Schuppenzelischicht (Cuticula) liegende Faser (Kortex) von der Färbung mit Pflanzenfarben nicht erfaßt wird. Die Cuticula die selbst schuppen- bzw. borkenförmig ausgebildet transparent und farblos ist, wird zwar durch die Pflanzenfarben geschlossen und erhält damit den gewünschten Glanz, die Nuancierung der Cuticula durch die Pflanzenfarben kann aber das Fehlen von Farbstoffen in der Kortex, wie es bei grauen Haaren der Fall ist, nicht abdecken.

Zum Abdecken grauer Haare verwendet man daher stets noch Oxidatiosfärbemittel, bei denen die farbgebenden Komponenten und Wasserstoffperoxid in die Kortex (Faserkern) des Haares hineinidiffundiert werden. Dies wird durch eine Quellung des Haares in alkalischer Lösung ermöglicht. Die eigentliche Färbung entsteht dann durch Oxidation. Dabei werden die Pigmentbildner im Innern des Kortex auf ein Vielfaches ihrer Einbringgröße vergrößert und bleiben somit im Kortex eingeschlossen. Nachteilig ist daran, zusätzlich zu den bereits erwähnten gesundheitsschädlichen Wirkungen, daß sowohl die Eigenpigmente des Haares als auch die Struktur der Schuppenzellenschicht und des Kortex dauerhaft verändert und beschädigt werden. Das Haar wird strohig und stumpf.

In der JP-A 61-143 315 ist ein zweistufiges Haarfärbeverfahren auf rein pflanzlicher Basis beschrieben, bei dem das Haar in einem ersten Schritt mit einer Paste aus gepulverten Hennablättern und in einem zweiten Schritt mit einer Paste aus gepulverten Henna- und Indigoblättern behandelt wird.

In der FR-A 1 173 973 wird ein Haarfärbeverfahren beschrieben, bei dem das Haar zunächst mit einer unter anderem ein Sulfid und einem Alizarin-Farbstoff enthaltenden Lösung behandelt wird und danach eine zweite Lösung auf das Haar aufgetragen wird, welche unter anderem einen Holzextrakt, Pyrogallol und ein Eisensalz enthält.

In der GB-A 2 093 868 wird ein Haarfärbemittel beschrieben, welches in einer einzigen Zusammensetzung vorliegt und unter anderem Pflanzenpulver (zum Beispiel Henna) und einen direktfärbenden Farbstoff enthält.

Demgemäß ist es Aufgabe der Erfindung, ein Verfahren zum Färben von Haaren, insbesondere von ergrauten Haaren, mit Pflanzenfarben zu schaffen, bei dem zusätzlich zur Nuancierung durch die Pflanzenfarben auch eine gute Farbdeckung, insbesondere bei grauen Haaren erreicht werden kann, jedoch ohne die Notwendigkeit der Verwendung in den Kortex eindringender Oxidationsfärbemittel.

Zur Lösung dieser Aufgabe sieht die Erfindung ein Verfahren zum Färben von Haaren vor, bei dem man zunächst das Haar durch Anfeuchten und Erwärmen quillt, sodann in einer üblichen Farbträgermasse einen nicht auf Pflanzenbasis beruhenden, nicht-oxidierenden kleinmolekularen direktziehenden Farbstoff auf das Haar aufträgt und 3 bis 20 Minuten lang einwirken läßt, anschließend einen Hennapflanzen enthaltenden Pflanzenbrei auf das Haar aufträgt und 10 bis 40 Minuten lang einwirken läßt und schließlich das Haar wäscht. Vorteilhafte Weiterbildungen der Erfindung sind in den Unteransprüchen definiert.

Das Wesentliche des Verfahrens ist also eine Zweistufigkeit. Im ersten Schritt erfolgt das Aufbringen nicht auf Pflanzenbasis beruhender, nicht-oxidierender kleinmolekularer direktziehender Farbstoffe, die in Anwesenheit von Feuchtigkeit (Nässe) und Wärme in die quellende Schuppenzellenschicht (Cuticula), nicht jedoch - wie die bekannten Oxidationsfärbemittel - in den Kortex eindringen.

Sie führen eine sog. semipermanente Färbung herbei, die je nach Konzentration des Farbstoffes und in Abhängigkeit von der Dauer der Einwirkung unter den genannten Bedingungen (Nässe, Wärme) eine bestimmte Farbtiefe und Farbintensität und damit eine Deckung des ergrauten Haares ergibt. Im zweiten Schritt werden die Pflanzenfarben aufgebracht, deren natürliche Wirkung infolge der auch im Henna enthaltenen Gerbsäure es ist, die Schuppenzellschicht (Cuticula) zu glätten und damit einerseits die im ersten Schritt in die Cuticula eingebrachten Farbstoffe dauerhaft einzuschließen und andererseits dem Haar - je nach Zusammensetzung der Pflanzenfarben - die gewünschte Nuance (rötlich, gold. aschfarben) zu verleihen, Der zweite Schritt bringt also bei Verwendung von Pflanzenfarben die Schließung der Cuticula mit sich.

Jeder der beiden Schritte ist in alleiniger Anwendung bekannt, führt jedoch zu einer nur wenig dauerhaften Färbung. Das Neue und Wesentliche der Erfindung ist die Kombination, die einerseits das natürliche Pigmente nicht mehr aufweisende Haar durch nicht-oxidierende Farbstoffe abdecken kann und andererseits durch die Einwirkung der Pflanzenfarben diese Abdeckung durch Schließen der Cuticula dauerhaft fixiert und gleichzeitig sowohl die gewünschte Nuancierung als auch die gewünschte Gättung des Haares ergibt.

Für die Farbstoffe, die in dem ersten Schritt aufgebracht werden, ist es wichtig, daß - wie bereits erwähnt - bei ihrer Anwendung bzw. Einbringung keine Oxidation erfolgt. Das Eindringen in den Kortex und die dort erfolgende Oxidationswirkung bekannter chemischer Färbemittel sollen gerade ausgeschlossen werden. Die bei der Erfindung verwendeten Farbstoffe sind an sich zur Tönung (semipermanente Färbung) bekannt. Eine geeignete Menge eines entsprechenden Wirkstoffs wird mit einer Trägermasse vermischt und auf das Haar aufgetragen. Unter einer geeigneten Menge des entsprechenden Wirkstoffs werden in der vorliegenden Anmeldung 1 bis 5 Gewichtsprozent, vorzugsweise 2 Gewichtsprozent, verstanden. Bei der Trägermasse kann es sich zum Beispiel um streichfähige Gele, Stärke, Lanolinderivate, Magnesiumcarbonate oder dergleichen handeln. Ein geeigneter Farbstoff sind zum Beispiel die unter der Marke "Arianor" (R) erhältlichen Farbstoffe mit folgender chemischer Struktur 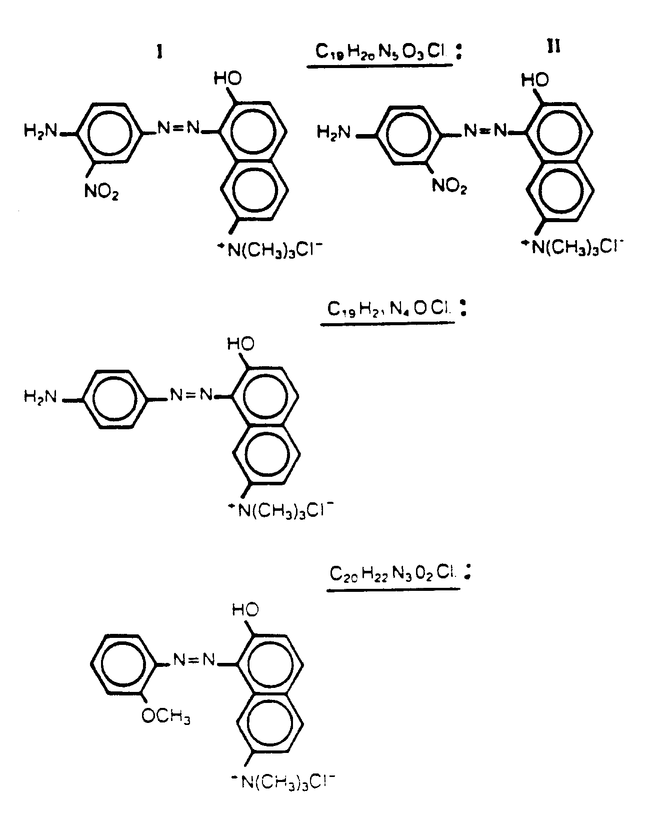
die sämtlich quarternäre Ammoniumgruppen enthalten. Der Träger wird mit dem Farbstoff und mit Wasser in einer Menge von ca. 30 ml angerührt und auf das Haar aufgetragen. Die Einwirkung unter Wärme erfolgt für die Dauer von 3 bis 15 Minuten, je nach gewünschter Farbtiefe und Farbintensität. Sie ist für die Farbdeckung verantwortlich.

Die danach aufgetragenen Pflanzenfarben werden je nach der gewünschten Farbnuance zusammengestellt. Dabei sollte immer bis zu einem gewissen Anteil Henna enthalten sein. Bei Henna handelt es sich um die gepulverten Blätter des Zyperstrauchs, auch Hennastrauch oder Mundholz (Lawsonia inermis od. L. alba), dessen für die Farbgebung wesentlicher Wirkstoff das Lawson (2-Hydroxy-1,4-naphthochinon) ist. Im allgemeinen ist Henna als ein rot bis orange färbender natürlicher Farbstoff bekannt; es gibt aber auch farblich neutrales Henna. Das ist darauf zurückzuführen, daß die Färbekraft des Henna mit dem Alter der abgeernteten Pflanzen abnimmt. Die Verwendung mindestens einer bestimmten Menge neutrales Henna sichert aber infolge der im Henna enthaltenen Gerbsäure-Komplexe bei einem pH-Wert von 4.5 die adstringierende Wirkung des zweiten Schrittes, die die Einschließung des im ersten Schritt aufgebrachten Farbstoffes durch Glättung und Zusammenziehen der Cuticula ergibt.

Andere Pflanzenfarben, die zur Erzielung bestimmter Farbnuancen beigemischt werden können, sind Kamille, Salbei, Lote, Wode, Indigo, Karschak, Vashma, Zwiebelschalen, Rhabarberwurzel, Schale der grünen Walnuss, usw. Diese Pflanzenfarben, die meist als Pulver vorliegen, werden in Wasser zu einem Pflanzenbrei angerührt und auf das Haar aufgetragen. Man läßt sie je nach gewünschter Nuancierung und nach Haareigenschaften 10 bis 40 Minunten einwirken. Dann wird das Haar leicht ausgewaschen. Damit ist der Färbevorgang beendet.

## Patentansprüche

1. Verfahren zum Färben von Haaren, bei dem man zunächst das Haar durch Anfeuchten und Erwärmen quillt, sodann in einer üblichen Farbträgermasse einen nicht auf Pflanzenbasis beruhenden, nicht-oxidierenden kleinmolekularen direktziehenden Farbstoff auf das Haar aufträgt und 3 bis 20 Minuten lang einwirken läßt, anschließend einen Hennapflanzen enthaltenden Pflanzenbrei auf das Haar aufträgt, 10 bis 40 Minuten lang einwirken läßt und schließlich das Haar wäscht.

2. Verfahren nach Anspruch 1, dadurch gekennzeichnet, daß der direktziehende Farbstoff in einer Menge von 1 bis 5 Gewichtsprozent enthalten ist.

3. Verfahren nach Anspruch 1 oder 2, dadurch gekennzeichnet, daß der direktziehende Farbstoff in einer Menge von 1 bis 2 Gewichtsprozent enthalten ist.

4. Verfahren nach einem der Ansprüche 1 bis 3, dadurch gekennzeichnet, daß der in dem ersten Schritt aufgetragene Farbstoff ein semipermanenter Farbstoff, dessen Wirkungsbestandteile quaternäre Ammoniumgruppen sind, ist.

5. Verfahren nach einem der Ansprüche 1 bis 4, dadurch gekennzeichnet, daß der Pflanzenbrei farblich neutrale Hennapflanzen enthält.

6. Verfahren nach einem der Ansprüche 1 bis 5, dadurch gekennzeichnet, daß der Pflanzenbrei 2-Hydroxy-1,4-naphthochinon enthält.

## Claims

1. Process for colouring hair, in which the hair is first soaked by moistening and warming, and a non-plant-based, non-oxidized, micro-molecular direct-acting dye substance in a conventional dye vehicle is then applied to the hair and allowed to act for 3 to 20 minutes, a vegetable paste containing henna plant material is subsequently applied to the hair and allowed to act for 10 to 40 minutes, and, finally, the hair is washed.

2. Process according to Claim 1, characterised in that the direct-acting dye substance is present in an amount of from 1 to 5 weight %.

3. Process according to Claim 1 or Claim 2, characterised in that the direct-acting dye substance is present in an amount of from 1 to 2 weight %.

4. Process according to any one of Claims 1 to 3, characterised in that the dye substance that was applied in the first step is a semi-permanent dye substance, the active ingredients of which are quaternary ammonium groups.

5. Process according to any one of Claims 1 to 4, characterised in that the vegetable paste contains henna plant material which is non-colouring.

6. Process according to any one of Claims 1 to 5, characterised in that the vegetable paste contains 2-hydroxy-1,4-naphthoquinone.

## Revendications

1. Procédé de coloration de cheveux, selon lequel on fait gonfler les cheveux par humidification et chauffage, puis on dépose sur les cheveux, dans une matière colorante usuelle, un colorant à action colorante directe de faible masse moléculaire non oxydant non à base de plantes, et on laisse agir pendant 3 à 20 minutes, on dépose sur les cheveux une pâte végétale contenant du henné végétal, on laisse agir pendant 10 à 40 minutes et finalement on lave les cheveux.

2. Procédé selon la revendication 1, caractérisé en ce que le colorant à action colorante directe est présent à raison de 1 à 5 % en poids.

3. Procédé selon la revendication 1 ou 2, caractérisée en ce que le colorant à action colorante directe est présent à raison de 1 à 2 % en poids.

4. Procédé selon l'une des revendications 1 à 3, caractérisé en ce que dans la première étape, le colorant déposé est une colorant semi-permanent dont les constituants actifs sont des groupes ammonium quaternaires.

5. Procédé selon l'une des revendications 1 à 4, caractérisé en ce que la pâte végétale contient du henné végétal neutre en coloration.

6. Procédé selon l'une des revendications 1 à 5, caractérisé en ce que la pâte végétale contient de la 2-hydroxy-1,4-naphtoquinone.
